Europäisches Patentamt

European Patent Office

Office européen des brevets

(11) Publication number: **0 047 480**
**B1**

(12) **EUROPEAN PATENT SPECIFICATION**

(45) Date of publication of patent specification: 05.02.86

(21) Application number: 81106848.5

(22) Date of filing: 02.09.81

(51) Int. Cl.⁴: **A 61 K 39/12**, A 61 K 9/50, C 12 N 7/06 // A61K39/145

(54) **Formation of an immunosome exclusively made of viral antigens reconstituted on an artificial membrane.**

(30) Priority: 05.09.80 US 184264

(43) Date of publication of application:
17.03.82 Bulletin 82/11

(45) Publication of the grant of the patent:
05.02.86 Bulletin 86/06

(84) Designated Contracting States:
BE DE FR GB

(56) References cited:
FR-A-2 325 387
FR-A-2 399 242
US-A-4 199 565
US-A-4 235 877

BIOLOGICAL ABSTRACTS, vol. 61, no. 8, 1976, page 4828, no. 46656, Philadelphia (USA); J.D. ALMEIDE et al.: "Formation of virosomes from influenza subunits and liposomes".

CHEMICAL ABSTRACTS, vol. 91, no. 19, 5th November 1979, page 233, no. 153174p, Columbus Ohio (USA); R.T.C. HUANG et al.: "Association of the envelope glycoproteins of influenza virus with liposomes - a model study on viral envelope assembly".

(73) Proprietor: INSTITUT ARMAND FRAPPIER
531 Boulevard des Prairies
Laval-des-Rapides Quebec H7V 1B7 (CA)

(72) Inventor: Thibodeau, Lise
3, Place Bellerive Apt 2304
Laval-des-Rapides Quebec H7V 1B2 (CA)
Inventor: Boudreault, Armand
12355 Jeanne-Mance
Montreal Quebec H3L 2C8 (CA)
Inventor: Naud, Pierre
444, rue de Nieul Apt 7
Laval-des-Rapides Quebec H7E 4B7 (CA)

(74) Representative: Casalonga, Axel et al
BUREAU D.A. CASALONGA OFFICE JOSSE &
PETIT Baaderstrasse 12-14
D-8000 München 5 (DE)

(56) References cited:
CHEMICAL ABSTRACTS, vol. 91, no. 9, 27th August 1979, page 231, no. 70422s, Columbus Ohio (USA); W.A. PETRI, Jr. et al.: "Reconstitution into liposomes of the glycoprotein of vesicular stomatitis virus by detergent dialysis".

Courier Press, Leamington Spa, England.

(56) References cited:
**CHEMICAL ABSTRACTS, vol. 90, no. 9, 26th
November 1979, page 175, no. 68173x,
Columbus Ohio (USA); T. UCHIDA et al.:
"Reconstitution of lipid vesicles associated
with HVJ (sendai virus) spikes. Purification and
some properties of vesicles containing
nontoxic fragment A of diphtheria toxin".**

## Description

This invention relates to a novel product which is exclusively made of viral antigens reconstituted on an artificial membrane known as a liposome. The new product will hereinafter be called an immunosome. It is understood that whenever it will be used in the present specification and in the appended claims, the term "immunosome" will be used according to the above definition.

The current influenza virus vaccines which are made of inactivated particles often induce undesirable local and general pyrogenic reactions. Therefore, there is a need for a vaccine that would not contain the substances responsible for these side effects.

In this line of thought, Almeida J. D. et al have done some work on a new product which they have called "virosome". The results have been published in an article entitled "Formation of Virosomes from Influenza Subunits and Lipozomes", which appeared in the Lancet, November 8, pp. 899—901, 1975.

The product called "virosome" was prepared by Almeida in the following manner: a mixture of lecitine and dicetyl-phosphate (9:1) is solubilized in chloroform, the organic solvent is evaporated under a flow of nitrogen; to the multilamellar lipidic film obtained a PB S buffer is added and the mixture is treated with ultrasonic waves for one hour and thirty minutes at a frequency of 50 KHz to produce closed vesicles. The viral subunits, i.e. hemagglutinines and neuraminidases are added to the suspension of liposomes and the mixture is treated with ultrasonic waves for 15 minutes to enable the integration of the subunits into the membrane. The results obtained are the following: there are viral subunits, in a small density, at the surface of some liposomes. The viral subunits seem to be anchored in the double sheet of phospholipidic material. No tests have been made to verify the hemagglutinating activity or the immunogenic power of these substances.

It could reasonably be assumed that the viral subunits are anchored both on the outside and on the inside of the liposome, specially in view of the particular manner of producing the so-called virosomes.

later, other searchers have tried to rebuild proteinic membranes of various viruses wrapped in liposomes. Among the most important are the following:

Davis J. P. P., Bucher D. J. "Integration of Purified Influenza Viral Neuraminidase with Liposomes". American Society of Microbiology, Annual Meeting Abstract T 114, 1980.

Helenius A., Fries E., Kartenbeck J. "Reconstitution of Semliki Forest Virus Membrane". J. Cell Biol. 75 pp. 866—880, 1977.

Hsu M. C., Scheid A., Choppin P. S. "Reconstitution of Membranes with Individual Paramyxovirus Glycoproteins and Phospholipid in Cholate Solutions". Virol. 95, pp. 476—491, 1979.

Huang R. T. C., Wahn K., Klenk H. D., Rott R.

"Association of the Envelope Glycoproteins of Influenza Virus with Liposomes. A Model Study on Viral Envelope Assembly". Virol. 97, pp. 212—217, 1979.

Miller D. K., Fever B. I., Vanderoef R., Lenard J. "Reconstituted G. protein-lipid Vesicles from Vesicular Stomatitis Virus and their Inhibition of VSV infection". J. Cell. Biol. 84, pp. 421—429, 1980.

Petri W. A., Wagner R. R. "Reconstitution into Liposomes of the Glycoproteins of Vesicular Stomatitis Virus by Detergent Dialysis". J. Biol. Chem. 254, 11, pp. 4313—4316, 1979.

It will be noted that our approach was totally different from the one used by Almeida. The basic method applied by everybody essentially consisted in solubilizing phospholipids and proteins in a detergent, to mix the two components together and to remove the detergent by dialysis. This procedure which consists in forming the liposome in the presence of proteins necessarily leads to the imprisonment of a large part of the proteins inside the liposome, which means that there is an important loss of immunogenic material.

It is an object of the present invention to synthetize a product called immunosome, which is a highly immunogenic, and is exclusively made of antigens attached on the membrane of a preformed liposome.

It is another object of the present invention to provide a method which is totally different from that of Almeida and other methods known in the art.

It is another object of the present invention to provide for a process of producing immunosomes by leaving out the technique used by Almeida which is characterized by solubilizing the phospholipids in an organic solvent which must be evaporated to give a multilamellar structure, and using ultrasonic waves which generally contribute to the destruction of the nature of the proteins.

It is another object of this invention to provide a method whereby the viral subunits are incorporated into the membranes by biochemical means.

It is still another object of the present invention to provide a method which uses preformed liposomes whereby glycoproteins are inserted at the surface of the liposomes by biochemical means.

It is well known that although the hemagglutinin and the neuraminidase induce immunity, when subunits HA (hemagglutinin) are in the presence of preformed liposomes, the incorporation into the phospholipidic double layer is carried out with an extremly low yield.

This difficulty can be overcome by providing a method of preparing immunosomes, as defined above, which comprises:

a) providing a quantity of preformed liposomes;

b) providing an antigen viral sub-unit-detergent complex;

c) treating the preformed liposomes to enable antigen subunits to anchor onto the liposomes so as to form projections on the outer surface of said liposomes; and

d) combining said antigen viral sub-unit-detergent complex with the liposomes obtained in step (c) so as to give said immunosomes.

According to the invention, the liposomes are prepared by solubilization of phospholipids in the presence of a dialysable detergent, followed by injection of the solution obtained in a buffer and dialysis.

In accordance with another preferred embodiment according to the invention, the antigen viral subunits are prepared by mixing a virus with a detergent to solubilize the membrane thereof, thereby giving a mixture of HA, neuraminidases, and a core formed of the nucleoprotein and proteins M, subjecting the mixture to centrifugation to separate the nucleo proteins and the proteins M therefrom and to give the HA and neuraminidases, subjecting the HA and neuraminidases to dialysis in an aqueous buffer solution and adding a detergent to give the protein detergent complex.

According to the invention, the viral sub-unit-detergent complex is added to liposomes which have been treated to become susceptible to anchor said sub-units and the mixture obtained is dialysed under a linearly decreasing gradient of detergent to give the immunosomes. If desired, when the gradient becomes zero, the suspension is dialysed in a PBS buffer.

The immunosome obtained comprises a membrane made of phospholipid material and viral subunits essentially distributed in the form of projections on the outer surface of the membrane, the immunosomes being substantially free of secondary effects when injected to a living being.

Figure 1 is a graph showing the immunogenicity of immunosomes produced by the method according to the invention, in comparison to viruses and viral subunits;

Figure 2 shows a group of immunosomes obtained by the method according to the invention; and

Figure 3 is an enlarged reproduction of an immunosome according to the invention showing the viral subunits anchored on the outer surface of the membrane.

It will be realized that whenever preformed liposomes are brought together with a solution of protein-detergent complex, the membrane of the liposomes has been treated to become so to speak more fluid such as by the addition of a small quantity of detergent.

The dialysis which can be carried out on a linearly decreasing gradient of detergent enables an exchange of the proteins of the protein-detergent complex in the phospholipidic layer of the liposome. It is therefore possible to artificially recreate a structure which is nearly identical to the viral particle, which possesses a very high hemagglutinating activity and which has been shown to be immunogenic.

The invention will now be illustrated by means of the following example.

Example

Formation of liposomes

— Phospholipids:phosphatidyl choline, cholesterol, lysolecitine (8:1:0.5) are solubilized in Tris-HCl buffer 10 mM pH about 7.4 which contains β - d - octylglucoside 30 mM.
— The solution of phospholipids is injected into a PBS buffer (saline phosphate) and the solution of liposomes so obtained is deposited in a small column made of Sephadex G-100® (hydrophilic insoluble molecular-sieve chromatographic medium, made by crosslinking Dextran).
— The column is centrifuged at 600 g for 15 seconds.
— The suspension of purified liposomes having a size between 30 and 90 nm is exhaustively dialyzed in PBS buffer.

Formation of protein-detergent complex

The HA and neuraminidases are in solution in PBS, concentration in proteins is adjusted to 700 µg/ml and to the solution of proteins, β - d - octylglucoside is added to give a final concentration of 7.5 mM: the mixture is allowed to stand at 22°C for about 10 minutes.

Formation of immunosome

To the suspension of liposomes β - d - octylglucoside in solution in Tris buffer (hydroxymethylaminomethane) 10 mM pH 7.4 is added to give a final concentration of 1 mM: the mixture is allowed to stand at 22°C for about 10 minutes.

To the suspension of liposomes made more fluid or in other words treated to become susceptible to anchor the viral subunits, the protein-detergent complex is added and the integration of the glycoproteins in the phospholipidic double layer of the liposome is carried out by dialysis on a linearly decreasing gradient of detergent.

— When the gradient becomes zero, the suspension of immunosomes is dialysed in a PBS buffer.

This procedure was carried out with both the A/England/864/75 (H3N2)X-49 and A/Bangkok/1/79 (H3N2)X-73 strains of the influenza virus.

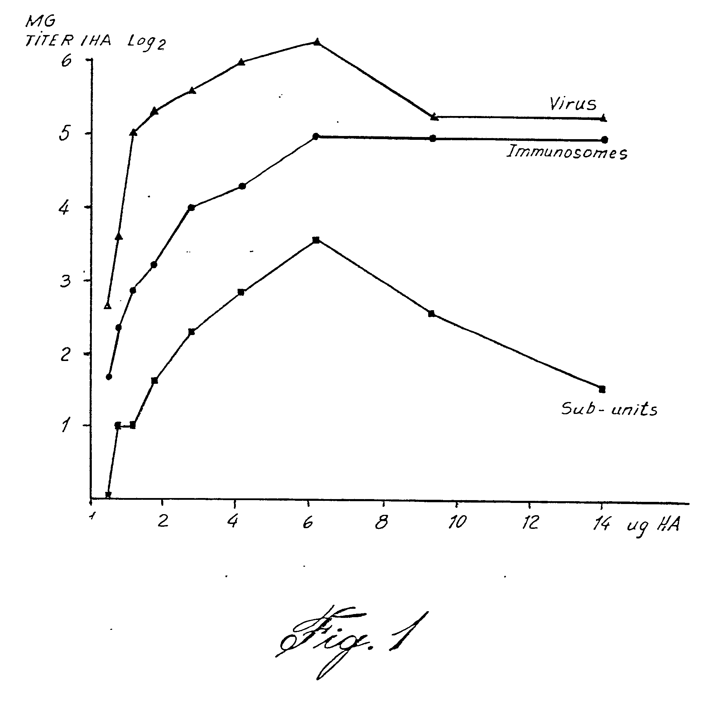

Tests were made to determine the activities of the sub-units and of the immunosome. Reference is made to the Table which follows and also Figure 1. It will be found that the immunosome according to the invention is much more active than the viral subunits and nearly as active as the whole virus.

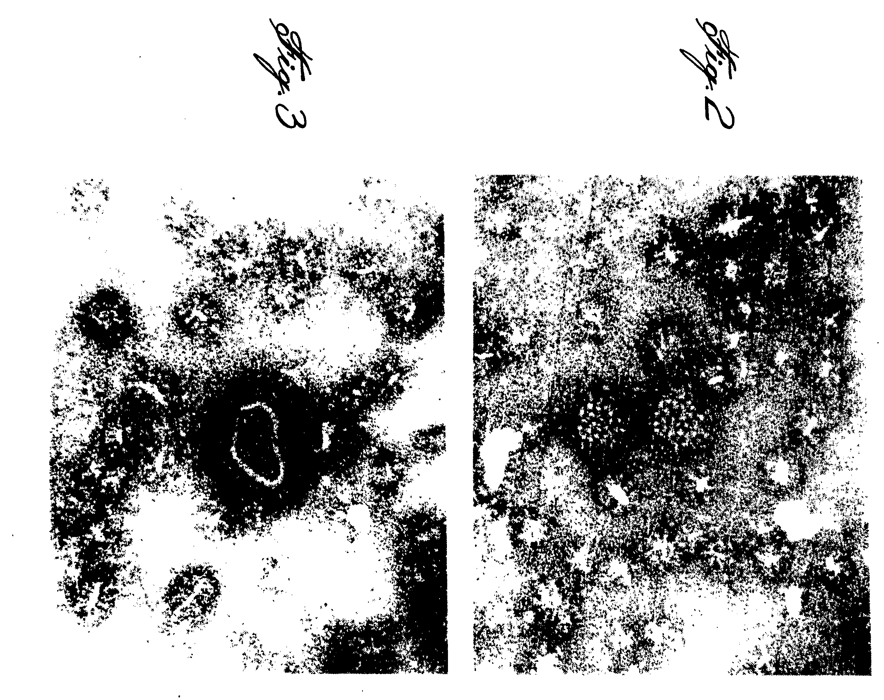

Referring to Figures 2 and 3 it will first of all be realized that the immunosomes can be obtained as a group of particles as shown in Figure 2.

Referring to Figure 3, it is obvious that the sub-units are exclusively and highly concentrated on the outer surface of the membrane and that inside the membrane, there are no nucleic acids or other substances which can give secondary effects upon injection.

HA activity of sub-units and of immunosome

| | µg protein in 50 µl | Titer* | Number of HA units in 50 µl | Specific activity **UHA/mg protein |
|---|---|---|---|---|
| Sub-units | 19 | $2^{12}$ | 4,096 | 215 500 |
| Immunosome | 6 | $2^{20}$ | 1,048 576 | 175 762 000 |

*Average of 15 determinations.
**Hemagglutinin units.

## Claims

1. A method of preparing immunosomes which comprises:

a) providing a quantity of preformed liposomes;

b) providing an antigen viral sub-unit-detergent complex;

c) treating the preformed liposomes to enable antigen sub-unit to anchor onto the liposomes so as to form projections on the outer surface of said liposomes; and

d) combining said antigen viral sub-unit-detergent complex with the liposomes obtained in step c) so as to give said immunosomes, characterized in that (i) said preformed liposomes are prepared by solubilization of phospholipids in the presence of a dialysable detergent, followed by injection of solution obtained in a buffer and dialysis, and (ii) that the antigen viral sub-unit-detergent complex obtained in step b) and the treated liposomes of step c) are dialyzed under a linearly decreasing gradient of dialyzable detergent to give said immunosomes.

2. A method according to claim 1, which comprises solubilizing phospholipids comprising a mixture of phosphatidyl choline, cholesterol and lysolecithin in a buffer containing as dialyzable detergent β - d - octylglucoside, injecting the solution in a buffer to form a suspension of liposomes and subjecting said suspension of liposomes to dialysis to give said preformed liposomes.

3. A method according to claim 2, wherein the phospholipids consist of a mixture of phosphatidyl choline, cholesterol and lysolecithin in a molar ratio of 8:1:0,5, said phospholipids are solubilized in a tris-HCl buffer containing about 30 mM β - d - octylglucoside and the solution is injected in a PBS buffer.

4. A method according to any preceding claims which comprises treating liposomes by chromatography to give liposomes which are substantially homogeneous and which have a size varying between 30 and 90 nm.

5. A method according to any preceding claims, wherein said antigen viral sub-units are prepared by mixing a virus with a dialyzable detergent to solubilize membrane thereof thereby giving a mixture of hemagglutinines, neuraminidases, nucleoproteins and proteins M, subjecting said mixture to centrifugation to separate said nucleoproteins and said proteins M therefrom and to give said hemagglutinines and neuraminidases, subjecting said hemagglutinines and neuraminidases to dialysis in an aqueous buffer solution and adding a dialyzable detergent to give said complex.

6. A method according to claim 5, wherein said dialyzable detergent is β - d - octylglucoside which is added in the form of a solution thereof in Tris-HCl buffer so as to give a final concentration of 7.5 mM, and allowing the mixture to stand at 22°C for about 10 minutes, thereby giving a protein-detergent complex susceptible to anchor in the membrane of the liposome.

7. A method according to any preceding claims, wherein when the gradient becomes zero, the suspension is dialyzed in a PBS buffer.

8. A method according to any preceding claims, wherein said liposomes treated to become susceptible to anchor said viral sub-units are prepared by adding to a solution of preformed liposomes a solution of β - d - octylglucoside in Tris-HCl buffer at pH about 7.4 so as to give a final concentration of 1 mM, and following the mixture obtained to stand at 22°C for about 10 minutes.

9. An immunosome comprising a membrane made of phospholipid material and viral sub-units essentially distributed in the form of projections on the outer surface of said membrane, said immunosome being prepared by a method according to claims 1 to 8 and being substantially free of secondary effects when injected to a living being.

## Revendications

1. Procédé de préparation d'immunosomes qui comprend:

a) la fourniture d'une quantité de liposomes préformés;

b) la fourniture d'un complexe détergent-subunité virale antigénique;

c) le traitement des liposomes préformés pour permettre à la sous-unité antigénique de se fixer sur le liposome de manière à former des projections à la surface externe desdits liposomes; et

d) la combinaison dudit complexe détergent-sous-unité virale antigénique avec les liposomes obtenus au stade c) pour obtenir lesdits immunosomes, caractérisé pr le fait que I) lesdits liposomes préformés sont préparés d'abord par la mise en solution de phospholipides en présence d'un détergent dialysable puis par l'introduction de la solution obtenue dans un tampon et de dialise, et II) que le complexe détergent-sous-unité virale antigénique obtenu au stade b) et les liposomes traités du stade c) sont dialysés avec un détergent dialysable avec un gradient décroissant de façon linéaire, pour donner lesdits immunosomes.

2. Procédé selon la revendication 1, qui comprend la mise en solution de phospholipides comprenant un mélange de phosphatidylcholine, de cholestérol et de lysolécithine dans un tampon contenant du β - d - octylglucoside en tant que détergent dialysable, l'introduction de la solution dans un tampon pour former une suspension de liposomes et la dialyse de ladite suspension de liposomes pour obtenir lesdits liposomes préformés.

3. Procédé selon la revendication 2, dans lequel les phospholipides consistent en un mélange de phosphatidylcholine, de cholestérol et de lysolécithine, en un rapport molaire de 8:1:0,5, lesdits phospholipides sont mis en solution dans un tampon Tris-HCl contenant environ 30 mM de β - d - octylglucoside et la solution est introduite dans un tampon PBS.

4. Procédé selon l'une quelconque des revendications précédentes, qui comprend le traitement des liposomes par chromatographie pour obtenir des liposomes qui sont pratiquement homogènes et qui ont unt taillie allant de 30 à 90 nm.

5. Procédé selon l'une quelconque des revendications précédentes, duvs lequel lesdites sout-unités virales antigéniques sont préparées en mélangeant un virus avec un détergent dialysable pour solubiliser le membrane de celui-ci, ce qui donne un mélange d'hémagglutinines, de neuraminidases, de nucléoprotéines et de protéines M, en soumettant ledit mélange à une centrifugation pour en séparer lesdites nucléoprotéines et lesdites protéines M et pour obtenir lesdites hémagglutinines et neuramidinases, en soumettant lesdites hémagglutinines et neuraminidases à une dialyse dans une solution tampon aqueuse et en ajoutant un détergent dialysable pour obtenir ledit complexe.

6. Procédé selon la revendication 5, dans lequel ledit détergent dialysable est le β - d - octyl-glucoside qui est ajouté sous la forme d'une solution de celui-ci dans un tampon Tris-HCl de manière à obtenir une concentration finale de 7,5 mM, et en laissant le mélange reposer pendant environ 10 minutes à 22°C, obtenant ainsi un complexe protéine-détergent capable de se fixer dans la membrane du liposome.

7. Procédé selon l'une quelconque des revendications précédentes, dans lequel lorsque le gradient atteint zéro, la suspension est dialysée dans un tampon PBS.

8. Procédé selon l'une quelconque des revendications précédentes, dans lequel lesdits liposomes traités pour être rendus capables de fixer lesdites sous-unités virales sont préparés en ajoutant à une solution de liposomes préformés une solution de β - d - octylglucoside dans un tampon Tris-HCl à pH d'environ 7,4 de façon à obtenir une concentration finale de 1 mM, et en laissant reposer le mélange obtenu pendant environ 10 minutes à 22°C.

9. Immunosome comprenant une membrane faite de substances phospholipidiques et de sout-unités virales essentiellement réparties sous forme de projections à la surface externe de ladite membrane, ledit immunosome étant préparé par un procédé selon les revendications 1 à 8 et étant pratiquement dépourvu d'effets secondaires lorsqu'injecté à un organisme vivant.

**Patentansprüche**

1. Verfahren zur Herstellung von Immunosomen, bestehend aus folgenden Schritten:
a) Beschaffung einer Menge vorbereiteter Liposomen;
b) Beschaffung eines Komplexes aus antigenen viralen Untereinheiten und Netzmittel;
c) Behandlung der vorbereiteten Liposome um die Verankerung der Antigen Untereinheit an den Liposomen zu ermöglichen, so dass Vorsprünge an der ausseren Oberfläche der genannten Liposomen gebildet werden, und
d) Kombinierung des genannten Komplexes aus antigenen viralen Untereinheiten und Netzmittel mit den im Schritt c) gebildeten Liposomen, so dass die genannten Immunosome erhalten werden, dadurch gekennzeichnet, dass (I) die vorbereiteten Liposome (i) durch Solubilisieren von Phospholipiden cn Anwesenheit eines dialysierbaren Netzmittels gebildet werden, die erhaltene Lösung in einen Puffer eingebracht wird und die so erhaltene Lösung dialysiert wird, und (ii) dass der Komplex von antigenen viralen Untereinheiten und Netzmittel erhalten im Schritt b) und die behandelten Liposome aus Schritt c) unter einem linear abfallenden Gradient von dialysierbarem Nitzmittel dialysiert werden um die genannten Immunosomen zu erhalten.

2. Verfahren nach Anspruch 1, wobei aus einem Gemisch von Phosphatidylcholin, Cholesterin und Lysolecithin bestehende Phospholipide in einem β - d - Octylglucosid als dialysierbares Netzmittel enthaltenden Puffer solubilisiert werden, die Lösung zur Bildung einer Suspension von Liposomen in einen Puffer eingebracht wird und diese Suspension aus Liposomen dialysiert wird um die vorbereiteten Liposomen zu erhalten.

3. Verfahren nach Anspruch 2, wobei die Phospholipide aus einem Gemisch von Phosphatidylcholin, Cholesterin und Lysolecithin in einem molaren Verhältnis von 8:1:0,5 bestehen, die genannten Phospholipide in einem Tris-HCl

Puffer enthaltend engefähr 30 mM β - d - Octyl-glucosid solubilisiert sind und die Lösung in eine mit Phosphat gepufferte physiologische Kochsalzlösung (PBS Puffer) eingebracht wird.

4. Verfahren nach einem der vorhergehenden Ansprüche, wobei die Liposome durch Chromatographie behandelt werden um im wesentlichen homogene und aufweisende Grösse aufweisende Liposome zu erhalten.

5. Verfahren nach einem der vorhergehenden Ansprüche, wobei die genannten antigenen viralen Untereinheiten durch Mischen eines Viruses mit einem dialysierbaren Netzmittel (um seine Membrane zu solubilisieren), hergestellt werden und dabei ein Gemisch aus Hämagglutininen, Neuraminidasen, Nucleoproteinen und Protein M erhalten wird, dieses Gemisch einer Zentrifugation unterworfen wird um die genannten Nucleoproteine und das genannte Protein M davon zu trennen und die genannten Hämagglutinine und Neuraminidasen zu erhalten, und diese beiden einer Dialyse in einer wässerigen Pufferlösung mit Zugabe eines dialysierbaren Netzmittels unterworfen werden um den genannten Komplex zu erhalten.

6. Verfahren nach Anspruch 5, wobei das dialysierbare Netzmittel β - d - Octylglucosid ist welches in der Form einer Lösung davon in Tris-HCl Puffer zugegeben wird um eine Endkonzentration von 7,5 mM zu ergeben und das Gemisch während 10 Minuten bei 22°C stehen gelassen wird um einen an der Membran des Liposoms verankerbaren Protein-Netzmittel Komplex zu erhalten.

7. Verfahren nach einem der vorhergehenden Ansprüche, wobei wenn der Gradient null geworden ist, die Suspension in einem PBS-Puffer dialysiert wird.

8. Verfahren nach einem der vorhergehenden Ansprüche, wobei die zur Verankerung der antigenen viralen Untereinheiten behandelten Liposome dadurch hergestellt werden, dass einer Lösung von vorbereiteten Liposomen eine Lösung von β - d - Octylglucosid in einem Tris-HCl-Puffer bei einem pH von ungefähr 7,4 zugegeben wird um eine 1 mM-Endkonzentration zu erhalten, und die erhaltene Mischung während ungefähr 10 Minuten bei 22°C stehen gelassen wird.

9. Ein Immunosome bestehend aus einer aus Phospholipidmaterial bestehenden Membran und aus viralen Untereinheiten die hauptsächlich in der Form von Vorsprüngen an der äusseren Oberfläche der genannten Membran verteilt sind, wobei die Immunosome durch ein Verfahren nach den Ansprüchen 1 bis 8 hergestellt sind und im wesentlichen keine sekundären Effekte hervorrufen wenn sie in ein lebendes Wesen injiziert werden.

Fig. 1

Fig.2

Fig.3